# EUROPEAN PATENT APPLICATION

(11) **EP 1 829 556 A1**
(43) Date of publication of application: **05.09.2007**
(21) Application number: 06119132.6
(22) Date of filing: 18.08.2006
(51) Int. Cl.: A61K 49/00, A61K 38/08

(54) **NK-3 Receptor in vivo assay**

(30) Priority: 29.08.2005 EP 05107892
(71) Applicant: F. HOFFMANN-LA ROCHE AG, 4070 Basel (CH)
(72) Inventor: Spooren, Will, 68130 Franken (FR)

(57) **Abstract**

The present invention relates to a method of assessing the in vivo potency of a NK3 receptor antagonist comprising: a) the non-human animal with the NK3 receptor antagonist of interest, b) treating an non-human animal with a NK3 R agonist treating, c) measuring the activity of the treated non-human animal, and d) correlating the effect of the antagonist of interest on the activity of the treated non-human animal with the in vivo potency of the antagonist.

SEQUENCE LISTING

<110> F. Hoffmann-La Roche AG

<120> NK-3 Receptor in vivo assay

<130> 23222

<160> 1

<170> PatentIn version 3.3

<210> 1
<211> 5
<212> PRT
<213> Unknown

<220>
<223> Description of artifical sequence: Consensus Sequence


<220>
<221> UNSURE
<222> (2)..(2)
<223> Xaa can be any naturally occurring amino acid

<400> 1

Phe Xaa Gly Leu Met
1               5

## Description

Tachykinins are a family of small peptides, e.g. Substance P, Neurokinin A and Neurokinin B, characterized by the shared presence of a common C-terminal amidated sequence Phe-Xaa-Gly-Leu-Met-NH2 (SEQ. ID. NO: 1). Their biological effects are mediated through three distinct types of receptors e.g. Neurokinin 1 (NK1), Neurokinin 2 (NK2) and Neurokinin 3 (NK3), respectively. Tachykinins and their receptors have been the focus of intense research over the past few years. Recently, the selective NK3-receptor (NK3-R) antagonist SR142801 (Osanetant) was shown to be active in a double blind placebo controlled clinical trial in schizophrenia patients (Meltzer H.Y., Arvanitis L., Bauer D., Rein W.: Aplacebo-controlled evaluation of four novel compounds for the treatment of schizophrenia and schizoaffective disorder. Am J Psych 161:975-84, 2004). However, the pharmacology and function of NK3 receptors in brain and behaviour and animal models are less well studied. There is a need for accurate means to assess the effect of NK3-R in vivo.

Therefore, the present invention provides a method of assessing the in vivo potency of a NK3 receptor antagonist comprising:
a) treating a non-human animal with the NK3-R antagonist of interest,
b) treating the non-human animal with a NK3-R agonist,
c) measuring the locomotor activity of the treated non-human animal and,
d) correlating the effect of the antagonist of interest on the locomotor activity of the treated non-human animal with the in vivo potency of the antagonist.

The term "in vivo potency" as used herein refers to the capacity of a compound, in this case a NK3-R antagonist, to produce the desired effect in a non-human animal, thereby attenuating the effect of NK3 or an NK3-R agonist to the NK3-R is the desired effect.

The term "NK3-R antagonist" or "antagonist" as used herein, refers to a compound capable of attenuating the effect of NK3 or an NK3-R agonist to the NK3-R.

The term "NK3-R agonist" or "agonist" as used herein refers to a compound capable of stimulating the Neurokinin 3 receptor.

The NK3 antagonist may be administered to the non-human animal by known enteral or parenteral routes, including but not limited to oral administration (such as oral gavage, sublingual administration or rectal administration), injection directly into the blood stream (such as intravenous or intra-arterial administration), or various parenteral routes (such as intraperitoneal and subcutaneous routes such as intramuscular), respiratory-based administration via an aerosol, and administration under the skin (i.e., transdermal, transcutaneous or percutaneous), as well as topical administration of the formulated compound of interest. A preferred form of administration of the antagonist is the oral (PO) administration or the intraperitoneal (IP) administration. Preferably, the antagonist is administered at a single dose.

The antagonist is typically administered some time prior to the testing of the activity of the non-human animal. The length of time depends of the time a compound needs for absorption and to overcome the blood-brain barrier. Preferably, the antagonist is administered to the non-human animal 15 to 240 minutes before the testing.

The NK3 receptor agonist may be any agonist of the Neurokinin 3 receptor, preferably the agonist is N-(3-carboxy-1-oxopropyl)-L-α-aspartyl-L-phenylalanyl-N-methyl-L-phenylalanylglycyl-L-leucyl-L-methioninamide (Senktide). The agonist maybe administered to the non-human animal by injection into the brain of the animal. More preferably, the agonist is injected into one or more ventricles. More preferably, the agonist is injected into the third (ventral) ventricle of the non-human animal. The agonist is typically administered at a single dose shortly prior to the testing of the activity of the non-human animal. For the injection of the NK3-receptor agonist into the brain, the non-human animal usually is anaestesized. Preferably, the testing starts right after the non-human animal has awaken, which may be 1 to 4 minutes after the administration of the NK3-R agonist.

The locomotor activity of the non-human includes but is not limited to walking, running, swimming or stereotypic activity such as e.g. grooming, licking or rearing. Preferably, the locomotor activity is measured by determining the distance covered by the non-human animal.

The non-human animal may be any animal excluding human. Preferably, the non-human animal is a rodent, more preferably a gerbil or a mouse. The most preferred non-human animal is a Mongolian gerbil (meriones unguiculates). The non-human animal may also be a non-human animal transgenic for the human NK3-receptor or the gerbil NK3-receptor.

Having now generally described this invention, the same will become better understood by reference to the specific examples, which are included herein for purpose of illustration only and are not intended to be limiting unless otherwise specified, in connection with the following figures.

### Figures:

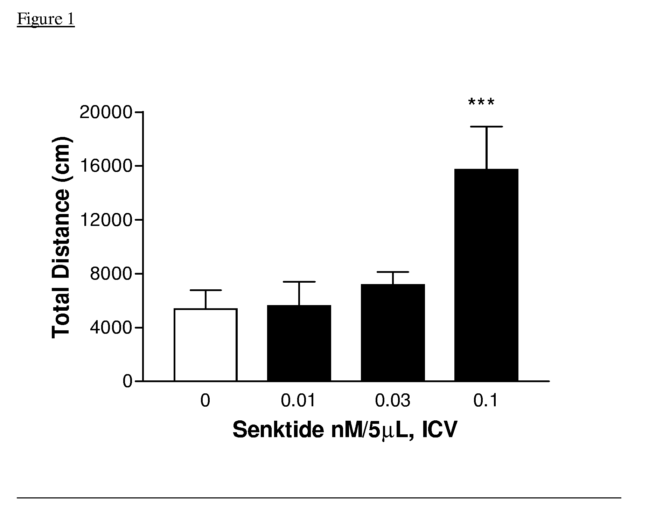

Figure 1 shows a graphical presentation of the locomotor activity induced by increasing doses of N-(3-carboxy-1-oxopropyl)-L-α-aspartyl -L-phenylalanyl-N-methyl-L-phenylalanylglycyl-L-leucyl-L-methioninamide (=senktide).
*** = p<0.001 (Dunnet multiple comparison test) versus vehicle.

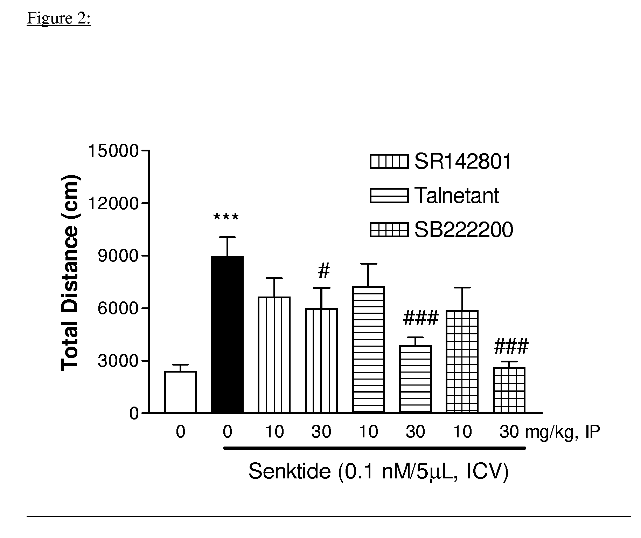

Figure 2 shows a graphical presentation of the effect of N-[1-[3-[(3R)-(1-benzoyl-3-(3,4-dichlorophenyl)-3-piperidinyl]propyl]-4-phenyl-4-piperidinyl]-N-methyl-acetamide (10 or 30 mg/kg, IP), 3-hydroxy-2-phenyl-N-[(1S)-1-phenylpropyl]-4-quinolinecarboxamide (10 or 30 mg/kg, IP) and 3-methyl-2-phenyl-N-[(1S)-1-phenylpropyl]-4-quinolinecarboxamide (10 or 30 mg/kg, IP) on locomotor activity induced by N-(3-carboxy-1-oxopropyl)-L-α-aspartyl-L-phenylalanyl-N-methyl-L-phenylalanylglycyl-L-leucyl-L-methioninamide.
# = p<0.05, ### = p<0.001(un-paired T-test) versus senktide.

### Example

Commercially available reagents referred to in the example were used according to manufacturer's instructions unless otherwise indicated.

### Materials and Methods

### Animals and housing

Male and female Mongolian gerbils [Biological Research Laboratories, 4414 Füllinsdorf, Switzerland] weighing between 40-70g were used. Gerbils were housed four per cage for approximately four to seven days following arrival in the laboratory colony. The gerbils had ad libitum access to tap water and lab chow in the home cage. The animal quarters were maintained on a 12:12 hour light-dark cycle with light onset at 6 a.m. and with both temperature (21-23°C) and humidity (55-65%) regulated. All testing was conducted during the light phase of the light-dark cycle. All procedures described here received prior approval from the City of Basel Cantonal Animal Protection Committee based on adherence to federal and local regulations. The experiments were carried out in an animal colony supervised by the veterinary staff of Hoffmann-La Roche Ltd.

### Compounds

### Angonist:

- N-(3-carboxy-1-oxopropyl)-L-α-aspartyl-L-phenylalanyl-N-methyl-L-phenylalanylglycyl-L-leucyl-L-methioninamide (Senktide)

### Antagonists:

- N-[1-[3-[(3R)-(1-benzoyl-3-(3,4-dichlorophenyl)-3-piperidinyl]propyl]-4-phenyl-4-piperidinyl]-N-methyl-acetamide (SR142801)
- 3-methyl-2-phenyl-N-[(1S)-1-phenylpropyl]-4-quinolinecarboxamide(SB222200)
- 3-hydroxy-2-phenyl-N-[(1S)-1-phenylpropyl]-4-quinolinecarboxamide (Talnetant)

### Preparation of drugs

The antagonists were prepared immediately prior to use (vehicle 0.3%Tween-80/saline). The antagonistic substances were administered IP or PO, 30min to 240min prior to testing. Senktide was dissolved in 0.1% BSAin distilled water and frozen (-20°C) in aliquots until use, where it was given by direct intracerebroventricular (i.c.v., ICV) injection.

### Inhibition of senktide-induced locomotor activity in gerbils

Gerbils were anaesthetised by inhalation of isoflurane/oxygen mixture, the scalp was exposed and Senktide (0.01 - 0.1 nmol/5µl) was administered into the third (ventral) ventricle (icv) via a cuffed 25G needle vertically inserted to a depth of 4.5mm below bregma. The incision was closed using a clip suture and gerbils were placed into perspex boxes on recovery of their righting reflex and then placed into locomotor activity boxes (Accuscan instruments, USA). Activity was then measured automatically for a period of 90 minutes.

### Statistical analysis

Data were analysed using a One way anova followed by Dunnet multiple comparison test or t-test (one-tailed) where appropriate (Statistica, Statsoft).

### Results

Senktide (0.01, 0.03 or 0.1 nM/5µ, ICV) induced a significant increase total distance traveled at a dose of 0.1 nM/5µL (ICV; Fig. 1).

SR142801 (10 or 30 mg/kg IP, 30min pre-treatment time) significantly reduced senktide (0.1nM/5µL, ICV)-induced locomotor activity at a dose of 30 mg/kg (Fig. 2).

Talnetant (10 or 30 mg/kg IP, 30min pre-treatment time) significantly reduced senktide (0.1nM/5µL, ICV)-induced locomotor activity at a dose of 30 mg/kg (Fig. 2).

SB222200 (10 or 30 mg/kg IP, 30min pre-treatment time) significantly reduced senktide (0.1nM/5µL, ICV)-induced locomotor activity at a dose of 30 mg/kg (Fig. 2).

Senktide induced a significant increase in locomotor activity as measured by means of total distance traveled over a period of 90 minutes following the ICV injection. This effect was inhibited by selective NK3 receptor antagonists such as SR142801, Talnetant and SB222200. These data indicate that senktide-induced locomotor activity is a selective NK3 receptor mediated effect and can be used to assess in vivo potency of NK3 receptor antagonists.

## Claims

1. An method of assessing the in vivo potency of a NK3 receptor antagonist comprising:
a) treating a non-human animal with the NK3 receptor antagonist of interest,
b) treating the non-human animal with a NK3 receptor agonist,
c) measuring the locomotor activity of the treated non-human animal and,
d) correlating the effect of the antagonist of interest on the locomotor activity of the treated non-human animal with the in vivo potency of the antagonist.

2. The method according to claim 1, wherein the NK3-R agonist is N-(3-carboxy-1-oxopropyl)-L-α-aspartyl-L-phenylalanyl-N-methyl-L-phenylalanylglycyl-L-leucyl-L-methioninamide.

3. The method according to claim 1 or 2, wherein the agonist is injected into the brain of the non-human animal.

4. The method according to claim 1 or 2, wherein the agonist is injected into one or more ventricles.

5. The method according to claim 1 or 2, wherein the agonist is injected into the third (ventral) ventricle.

6. The method according to any one of the claims 1 to 5, wherein the non-human animal is a rodent.

7. The method according to any one of the claims 1 to 5, wherein the non-human animal is a gerbil or a mouse.
